# EUROPEAN PATENT APPLICATION

(11) **EP 0 526 866 A1**
(43) Date of publication of application: **10.02.1993**
(21) Application number: 92113213.0
(22) Date of filing: 03.08.1992
(51) Int. Cl.: A61K 31/70

(54) **The use of adenosine compounds for the preparation in the treatment of ischaemia**

(30) Priority: 07.08.1991 IT MI912224
(71) Applicant: BIORESEARCH S.p.A., I-20060 Liscate (Milano) (IT)
(72) Inventor: Bassi, Luigi, I-26027 Rivolta d'adda (Prov. of Cremona (IT); Gennari, Federico, I-20060 Trucazzano (Prov. of Milano) (IT)
(74) Representative: Gervasi, Gemma, Dr.

(57) **Abstract**

The use of adenosine compounds chosen from the group consisting of 5'-deoxy-5'-methylthioadenosine, 5'-deoxy-5'-isobutylthioadenosine, 5'-deoxy-5'-benzylthioadenosine, and 5'-deoxy-5'-methylthio N⁶ methyladenosine, for the preparation of pharmaceutical compositions for the treatment of forms of ischaemia.

## Description

### PRIOR ART

Some adenosine compounds are known which are used in various fields of pharmacology.

For example, 5'-deoxy-5'-methylthioadenosine is amply described in US 4.454.122 and US 4.373.097 patents by the applicant who highlights the pharmacological uses of said compound as an anti-inflammatory, anti-pyretic, anticoagulant of platelets and as sleep inducing agent. Alkylthioethers of 5'-deox- yadenosine are also described in patent GB 1.555.991 as anti-viral and cytostatic agents, and as inhibitors of oncogen transformation in tissues.

### SUMMARY

It has now been found, and is the object of the present patent, that adenosine derivatives having the general formula: 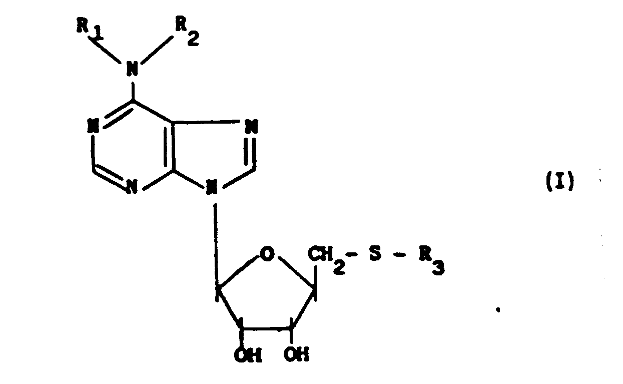
in which:
R₁ = R₂ = H and R₃ = methyl, isobutyl, benzyl
or R₁ = H and R₂ = R₃ = methyl,
have anti-ischaemic properties.

The present invention thus refers to the use of adenosine derivatives with general formula (I) for the preparation of pharmaceutical compositions for the treatment of ischaemia.

### DETAILED DESCRIPTION OF THE INVENTION

The characteristics of the compounds that are the object of the invention when used in the treatment of ischaemia are further set out in the detailed description that follows.

The compounds of the present invention suitable for the preparation of pharmaceutical compounds for the treatment of ischaemia are as follows:
- 5'-deoxy-5'-methylthioadenosine (MTA)
- 5'-deoxy-5'-isobutylthioadenosine (BR 300)
- 5'-deoxy-5'-benzylthioadenosine (BR 203)
- 5'-deoxy-5'-methylthio N⁶ methyladenosine (BR 230).

Their anti-ischaemic effect was studied on an isolated and reperfused rabbit heart model in which ischaemia was induced by reducing the coronary flow according to the method described by C. Ceconi, S. Curello, A. Cargnoni, R. Ferrari, A. Albertini, and O. Visioli in: The role of Glutathione status in the protection against ischaemic and reperfusion damage: effect of N-Acetyl Cysteine (J. Mol. Cell Cardiol., 1988: 20, 5-13).

The animal was killed by cervical dislocation, and its cardiac muscle was quickly removed and immersed in an oxygenated Krebs-Henselait solution at 37 ° C.

Once the fat and connecting tissues were removed, the pulmonary artery was incannulated and by perfusing the organ according to the Landerdorff non-recycling method with a modified Krebs-Henselait (mM: NaCl 115; NaHC0₃ 25; KCI 4; KH₂ PO₄ 0.9; MgS0₄ 1.1; CaC1₂ 1.5; Glucose 11) continuously carboxygenated solution (95% 0₂ + 5% C0₂) at a temperature of 37° C.

The right atrium was removed so as to avoid over-high frequencies, and the Krebs-Henselait solution was introduced at a constant flow of 24 ml/min. using a peristaltic pump (Miniplus-Gilson).

Ventricular pressure was measured using a latex bag connected to a pressure transducer and inserted in the left ventricle.

The bag was inflated with distilled H₂0 until it had a diastolic pressure of 5-10 mmHg.

Cardiac frequency was detected from the ventricular pressure trace. After 15 minutes of settling down at its own spontaneaous beat, the heart was electrically stimulated to 180 beats/min. using impulses with the following characteristics: frequency 0.33 sec., delay 0.01 msec., duration 0.3 sec., interval 0.3 sec., width 3 msec., volts 5-10 (Mangoni BM ST3 stimulator).

After 15 minutes of equilibration, the heart was perfused with Krebs-Henselait solution at 24 ml/min in an aerobic condition for 30 minutes, then made ischaemic by reducing the flow from 24 ml/min. to 1 ml/min for a period of 90 minutes, and finally reperfused with 24 ml/min. for a further 30 minutes.

The three stages of perfusion, ischaemia, and reperfusion shown in the diagrams on the attached figures are respectively from -30 to 0, from 0 to 90, and from 90 to 120 minutes.

Throughout the experiment the temperature of the cardiac muscle (36-37 C) was controlled using a probe (Termist) inserted through the ascending aorta.

In all the experiments, the compounds being tested were initially dissolved in an aqueous solution of HCI, and then diluted with Krebs-Henselait solution until the desired concentration was reached (from 10-⁴ to 10-⁶ M). This solution was then perfused for the entire duration of the experiment (30 minutes perfusion, 90 minutes ischaemia, and 30 minutes reperfusion).

The respective control subjects were perfused with a Krebs-Henselait solution containing a hydrochloric acid concentration equivalent to that present in the compound solutions and equimolar to the concentration of the active substance (from 10-⁴ M to 10-⁶ M). All the compounds listed at the start of this description were found to be active in this model of cardiac ischaemia, the activity being demonstrated by the recovery of the difference between systolic and diastolic pressure following reperfusion, this difference being a measure of cardiac contractility.

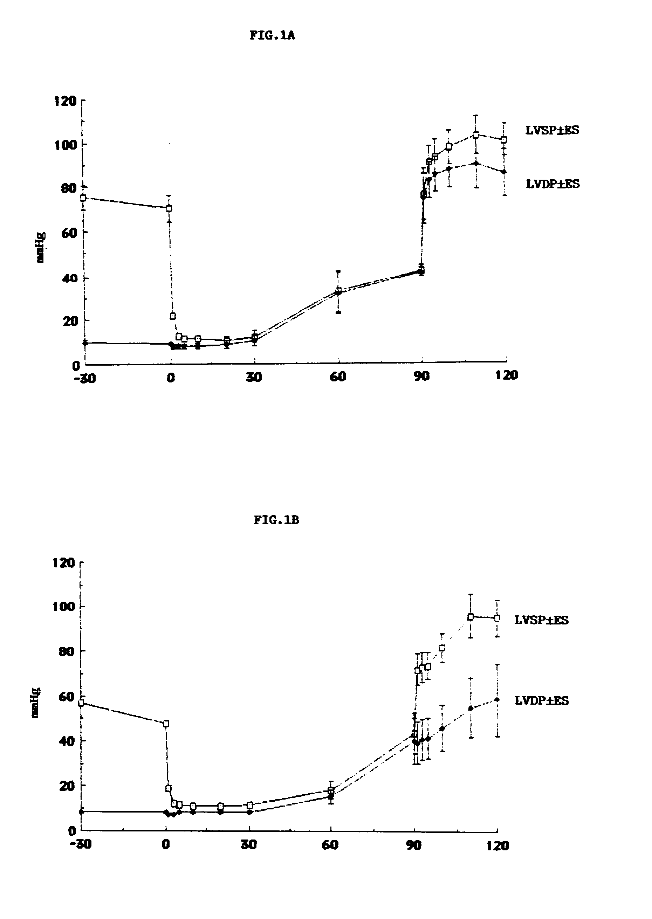

The results of the experiments are shown in the diagrams in figures 1A, 1B, 2A, 2B, 3A, and 3B, in which the time in minutes is shown along the X axis and left ventricle pressure in mm/Hg is shown on the Y axis.

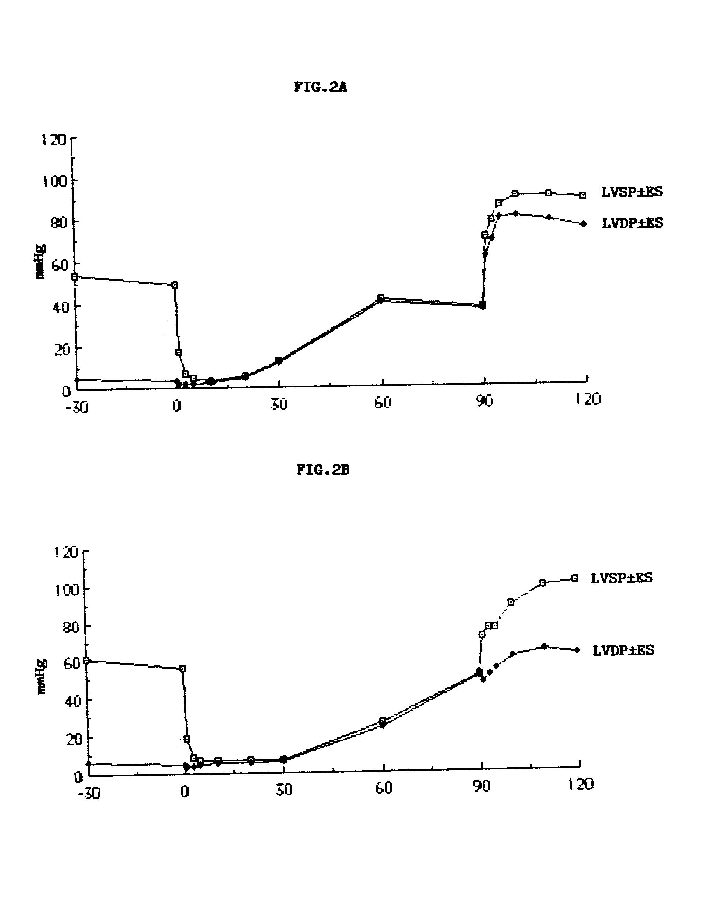

Figures 1 B and 2B show the results of experiments respectively using 5'-deoxy-5'-methylthioadenosine (MTA) and 5'-deoxy-5'-methylthio N⁶ methyladenosine (BR 230) at a concentration of 10-⁵ M, and figures 1A and 2A show the results of control experiments carried out without using these substances.

The average ± ES of the diastolic pressure (LVDP) and systolic pressure (LVSP) values are also shown on the diagrams (3 experiments).

In figure 1A, the ischaemic condition (starting at time 0 mins.) induces a rapid decline in systolic pressure until it reaches a level close to diastolic pressure, and the contractile activity completely ceases a few minutes after the onset of ischaemia. During the ischaemic stage (from 0 to 90 mins.) there is a gradual increase in diastolic and systolic pressure which, once the coronary flow is restored (after 90 mins.), dramatically and rapidly increases, and diastolic pressure in particular reaches levels close to systolic pressure levels thus seriously compromising cardiac contractility. It can be calculated from the diagrams that the average difference between systolic and diastolic pressure at the reperfusion stage is 24% of the average difference at the pre-ischaemia stage.

The average LVDP and LVSP levels for the 3 hearts perfused with a solution containing MTA are shown in figure 1 B.

In this case, diastolic pressure at the ischaemia stage rises more slowly than systolic pressure, and remains at a level far below the levels reached in the control experiment (fig. 1A) at the reperfusion stage. At this stage, the difference between systolic and diastolic pressure was 92.1 % of the average value of the same difference at the pre-ischaemia stage.

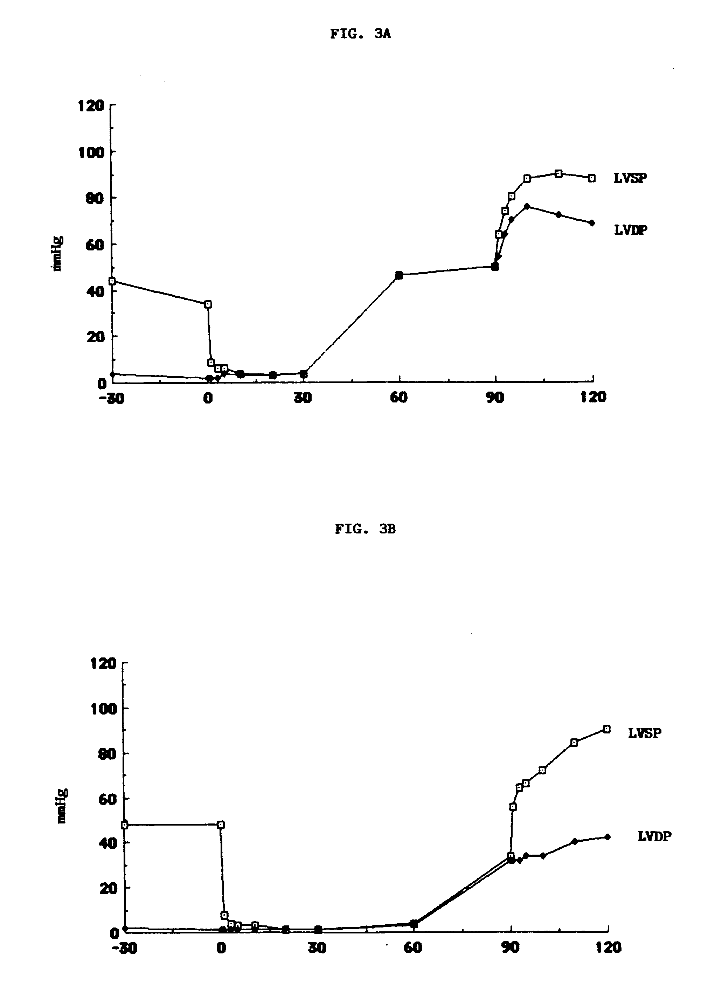

The values obtained with perfusion using BR 230 (an average of 3 experiments) are shown in figure 2B. When compared with the respective control values shown in figure 2A, these values are seen to be similar to those obtained using a perfusion of MTA. The difference between systolic and diastolic pressure at the reperfusion stage using BR 230 (figure 2B) is 75% of the same difference at the pre-ischaemia stage, whereas in the control experiments (figure 2A), this difference was 30%. Figure 3B shows the diagram of an experiment carried out using BR 230 10-⁶ M. Figure 3A shows the relative control experiment. The compound showed a protective effect even at this concentration.

The results obtained using the described experimental model show that the adenosine compounds studied are capable of protecting against ischaemic damage and reperfusion injury, and are thus suitable for therapeutic use in various ischaemia situations, such as cases of cerebral or myocardial ischaemia, or for ischaemia of any type of organs or tissues.

The present invention thus refers to the use of adenosine compounds with general formula (I) for the preparation of pharmaceutical compositions for the treatment of forms of ischaemia.

These compositions contain pharmacologically active quantities of an adenosine compound with general formula (I), and are formulated with pharmacologically acceptable solvents, dilutants, and excipients, such as sterile and apyrogenic water in hypodermic solutions, or maize starch, lactose, silica, microcrystalline cellulose in oral formulations.

In the aforementioned compositions the concentration of the adenosine compound can be from 50 to 500 mg.

They are administered parenterally or orally and their daily dose is between 50 and 1000 mg.

## Claims

1. Use of adenosine derivatives with the general formula (I) 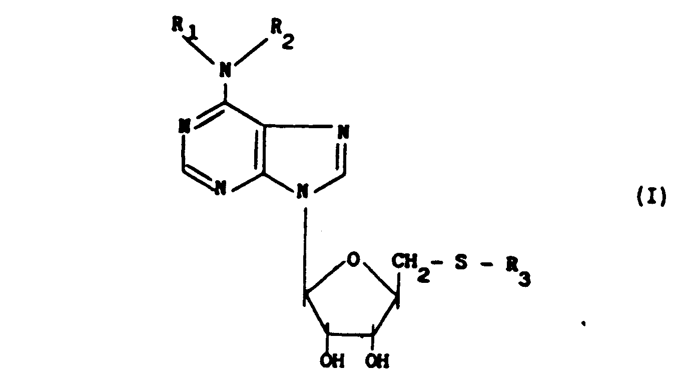
in which:
R₁ = R₂ = H and R₃ = methyl, isobutyl, benzyl or R₁ = H and R₂ = R₃ = methyl,
as the active principle for the preparation of pharmaceutical compositions for the treatment of ischaemia.

2. The use as in Claim 1, characterized in that the aforementioned adenosine compound is 5'-deoxy-5'-methylthioadenosine.

3. The use as in Claim 1, characterized in that the aforementioned adenosine compound is 5'-deoxy-5'-isobutylthioadenosine.

4. The use as in Claim 1, characterized in that the aforementioned adenosine compound is 5'-deoxy-5'-benzylthioadenosine.

5. The use as in Claim 1, characterized in that the aforementioned adenosine compound is 5'-deoxy-5'-methylthio N⁶ methyladenosine.

6. The use as in Claim 1, characterized in that the aforementioned compositions have a adenosine compound concentration of between 50 mg and 500 mg and comprise pharmacologically acceptable solvents, dilutants, and excipients consisting of maize starch, lactose, silica, and microcrystalline cellulose.
